(19)

**Europäisches Patentamt**
**European Patent Office**
**Office européen des brevets**

(11) **EP 4 730 304 A1**

(12) **EUROPEAN PATENT APPLICATION**

(43) Date of publication:
22.04.2026 Bulletin 2026/17

(21) Application number: 25208757.2

(22) Date of filing: **15.10.2025**

(51) International Patent Classification (IPC):
*G09B 21/00* (2006.01)  *G06T 5/00* (2024.01)
*G06T 7/00* (2017.01)  *A61B 3/00* (2006.01)
*A61B 3/02* (2006.01)  *A61B 3/032* (2006.01)
*A61B 3/08* (2006.01)

(52) Cooperative Patent Classification (CPC):
G09B 21/001; A61B 3/0033; A61B 3/005;
A61B 3/0058; A61B 3/022; A61B 3/028;
A61B 3/032; A61B 3/08; G06T 5/00; G09B 21/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **15.10.2024  IT 202400022926**

(71) Applicant: **New Digital Consulting S.r.l. - Società Benefit**
**84134 Salerno (IT)**

(72) Inventor: **SANGIUOLO, Raffaele**
**84134 SALERNO (IT)**

(74) Representative: **Baldi, Claudio**
**Ing. Claudio Baldi S.r.l.**
**Viale Cavallotti, 13**
**60035 Jesi (Ancona) (IT)**

(54) **METHOD FOR GENERATING AND DISPLAYING A 3D FILM THAT REPRODUCES A USER'S VISION**

(57)    Method for generating and displaying a video that simulates a user's vision; said method comprising an assessment step (F1) wherein a plurality of tests (T, T1, T2, T3, T4) are executed on the user to evaluate the user's visual abilities and information and indices (Q, Q1, Q2, Q3; L1, L2, L3, L4....) on the user's visual abilities are collected, an acquisition step (F2) wherein a plurality of sequential images of the same scene (H) are acquired from said two different positions, so as to have a plurality of right and left images (D1, D2, D3..... S1, S2, S3....), a post-processing step (F3) wherein said right images (D1, D2, D3.....) and said left images (S1, S2, S3....) are processed based on said information and indices (Q, Q1, Q2, Q3, L1, L2, L3, L4....) detected in said evaluation step (F1), so as to obtain aberrated binocular images (V1b, V2b, V3b), and a video playback step (F4) wherein said aberrated binocular images (V1b, V2b, V3b) are displayed on a display (200) of a visor (2) so as to play-back said video (V) emulating a user's vision.

FIG. 1

**Description**

The present patent application for industrial invention relates to a method for generating and displaying a 3D video reproducing a user's vision.

As it is known, sight is one of the five senses responsible for perceiving visual stimuli, such as light, shapes, and colors. Human beings have a binocular vision that allows them to see with both eyes simultaneously and perceive the space and three-dimensionality of the surrounding world.

If impaired by disease or disability, vision can dramatically affect people's lifestyle and quality of life, as well as their perception of reality and the world around them.

If it were be possible to "see" in the same way as a partially-sighted user or a visually impaired user, it would be easier to make user assessments.

Currently, there are no systems or technologies that replicate each person's actual vision.

Such a system, capable of replicating a user's vision, would have positive implications in all those fields of application that require assessing and understanding how a person really sees the world around him/her.

The method according to the invention has no precedent in the prior art since, to date, vision assessments are only made using numerical indices that do not allow to effectively understand how a user sees or does not see the world around him/her.

Therefore, there is currently an unmet need related to the fact that it is only possible to imagine how a visually impaired person sees, but it is impossible to verify and experience with one's own eyes how these people actually perceive the world around them.

The purpose of this invention is to provide a solution to such a need by devising a method for generating and displaying a 3D video that emulates a user's vision.

Such a purpose is achieved in accordance with the invention with the features listed in the attached independent claim 1.

Advantageous embodiments appear from the dependent claims.

The method according to the invention is defined by claim 1.

For greater clarity, the description of the method according to the invention continues with reference to the attached drawings, which are for illustrative purposes only and not limiting, wherein:

Fig. 1 is a block diagram illustrating a system for implementing the method according to the invention;
Fig. 1A is a flowchart illustrating the method according to the invention;
Fig. 2 is an axonometric view of a visor of the system of Fig. 1 supported by a supporting frame;
Fig. 3 is a schematic view of the elements of the visor of Fig. 2;
Fig. 4 are flowcharts illustrating a set of preliminary tests of the method according to the invention;
Figs. 5 and 5A are two front views of the two eyepieces and of the display screen during an assessment of the monocular visual acuity for the right eye;
Figs. 6 and 6A are two front views of the two eyepieces and of the display screen during an assessment of the monocular visual acuity for the left eye;
Figs. 7 and 7A are two front views of screens of the visor display during a binocular visual acuity assessment;
Figs. 8 and 8A are two front views of screens of the visor display during a contrast sensitivity assessment;
by way of example, Fig. 9 shows two patterns displayed on the visor display, in front of the two eyepieces, used for a stereoscopy assessment;
Fig. 9A shows how a user perceives the two patterns displayed on the visor display;
Fig. 10 is a view of a display screen wherein analysis points for evaluating a binocular field of view are indicated;
Figs. 10A, 10B, 10C, and 10D are four views of screens of the visor display during the execution of a test to assess the binocular field of view;
Fig. 11 is a diagrammatic axonometric view of a shooting step of a scene of the method according to the invention;
Fig. 12 is a block diagram illustrating a control unit of the system of Fig. 1;
Fig. 13 is a flowchart of a post-processing step of the method according to the invention;
Figs. 14, 15, 16, 17, and 18 are schematic views of the image processing steps to which the images acquired in the shooting step are subjected.

With reference to the attached figures, a method for generating and displaying a video simulating the vision of a visually impaired user is described.

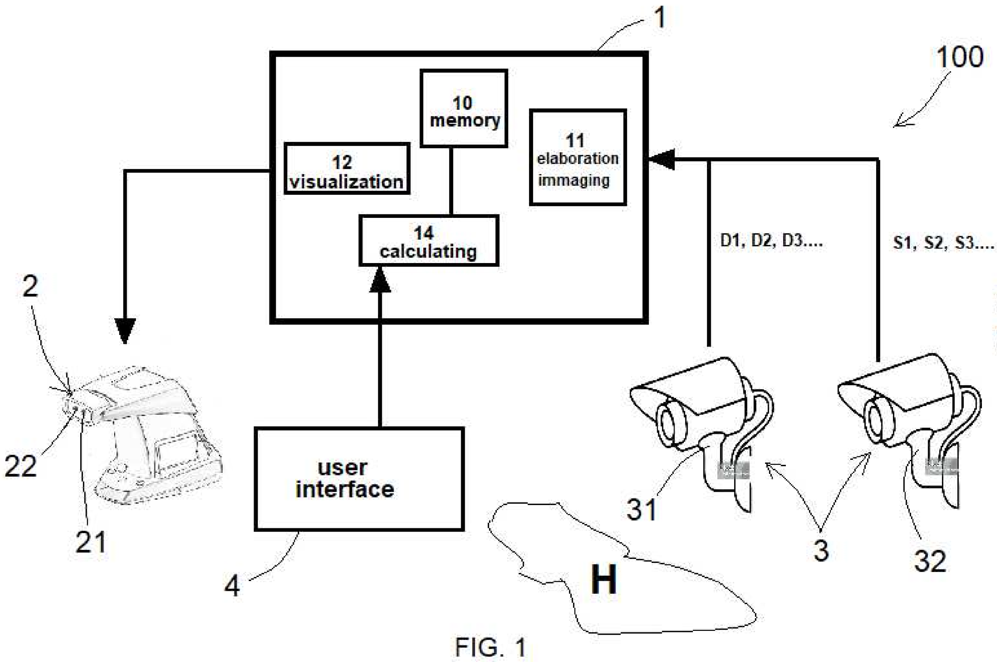

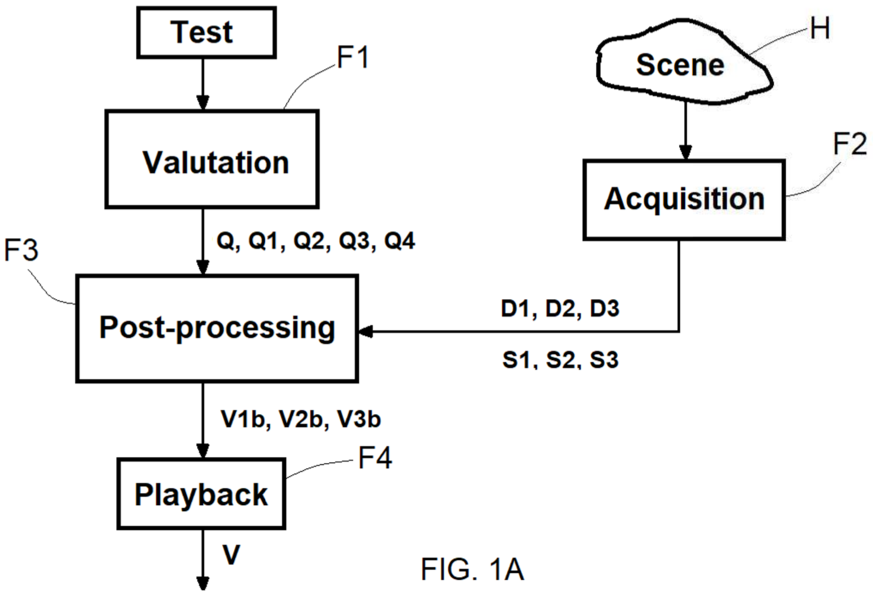

With reference to Fig. 1, the method according to the invention is implemented by means of a system (100) comprising: a visor (2), a user interface (4), image acquisition means (3), and a control unit (1).

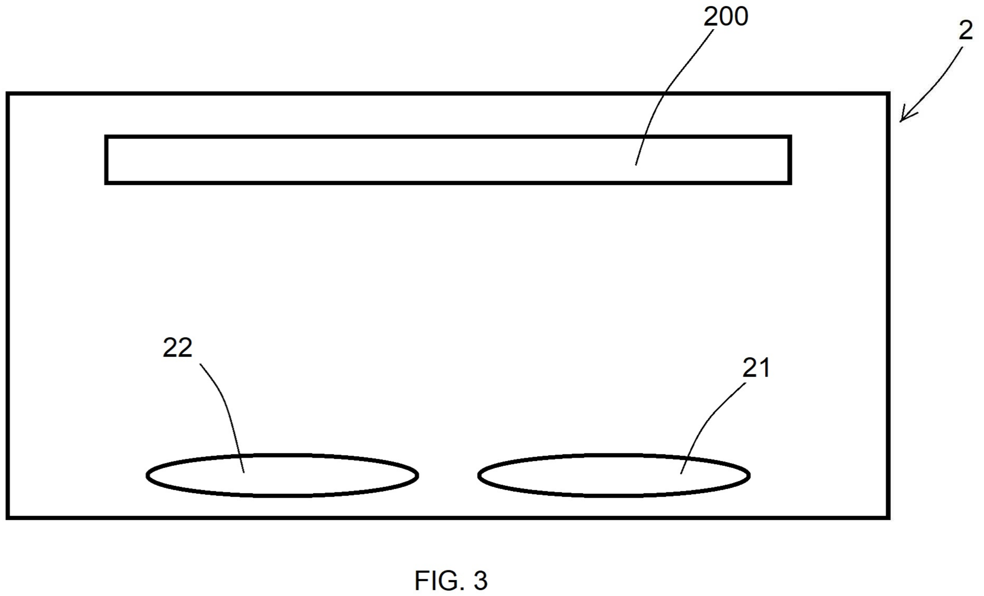

With reference to Figs. 1, 3, and 4, the visor (2) is a visor suitable for being used by a user. The visor (2) comprises a right eyepiece (21), a left eyepiece (22), and a display (200) for displaying images and videos, wherein the left eyepiece (22) of the visor (2) is suitable for being used for the perception of the user's left eye, whereas the right eyepiece (21) of the visor (2) is suitable for being used for the perception of the user's right eye. During use, the user places his/her face in front of the visor, resting his/her right eye on the right eyepiece (21) and his/her left eye on the left eyepiece (22) so that his/her eyes can see the display (200) of the visor (2). The visor (2) is supported by a frame (9) comprising a column (91) and a base (92).

The visor (2) may further comprise optical sensors and/or cameras suitable for detecting the patient's pupils, biconvex lenses, illuminators, and an interpupillary distance adjustment system.

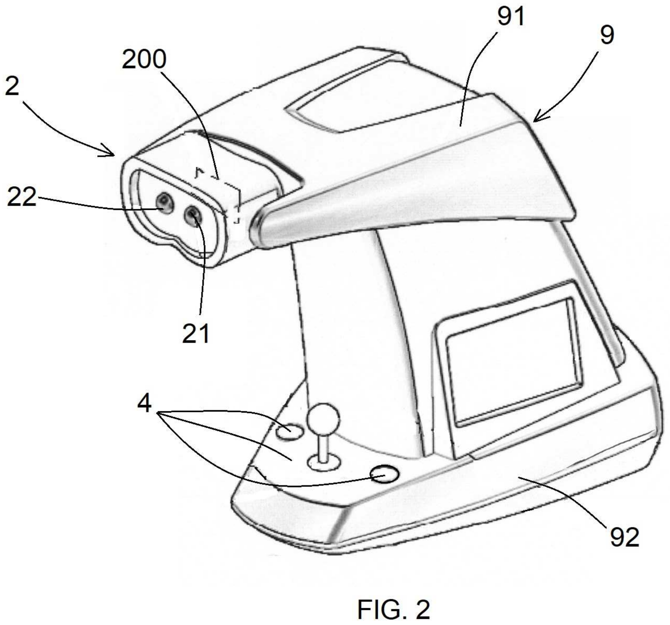

The user interface (4) comprises a joystick and/or keys and/or microphones configured to detect inputs (IN) from the user during the execution of tests (T, T1, T2, T3, T4) performed by the user using the visor (2). The joysticks and/or keys of the user interface (4) can be mounted on the base (92) of the frame (9), as shown in Fig. 2.

With reference to Fig. 1, the image acquisition means (3) are set up and configured to acquire images (D1, D2, D3.....; S1, S2, S3) of a scene (H) from two different positions, so as to simulate the vision of a right eye and of a left eye. The image acquisition means (3) comprise a right camera (31) that acquires and reproduces the vision of a right eye, and a left camera (32) that acquires and reproduces the vision of a left eye.

The visor (2), the user interface (4), and the image acquisition means (3) are operatively connected to the control unit (1).

The control unit (1) comprises an image processing software (11), a memory (10), calculation means (14), and a viewing software (12).

The image processing software (11) is configured to process the images received from the image acquisition means (3).

The viewing software (12) is configured so that the internal display (200) of the visor can display a screen with a background and symbols and/or images and/or videos.

The calculation means (14) are configured to receive the inputs (IN) from the user interface (4) during the execution of the tests (T, T1, T2, T3, T4) in order to calculate an evaluation, information, or visual index relative to each test (T, T1, T2, T3, T4) performed by the user.

The control unit (1) further comprises a graphics card and connections, such as video, Wi-Fi, USB, LAN inputs and outputs, and the like.

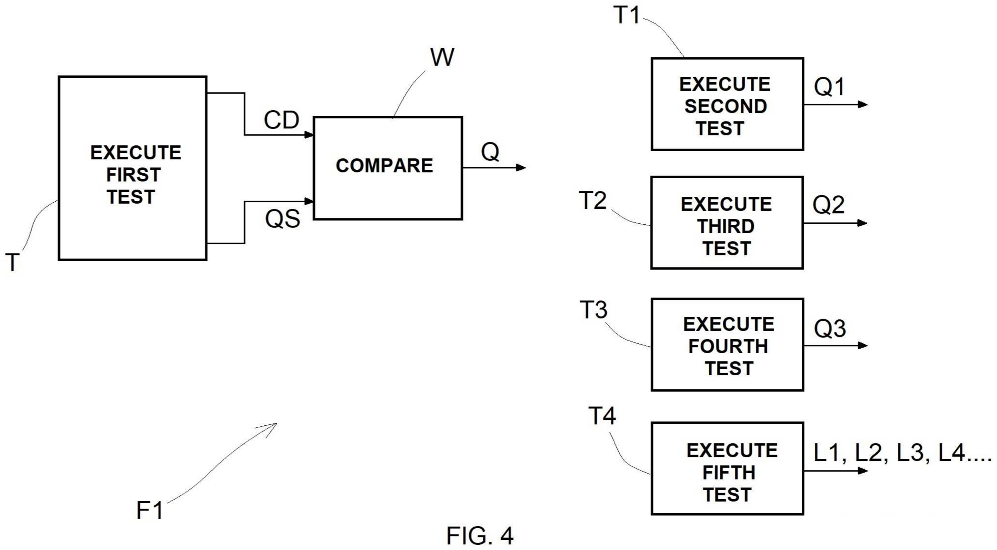

With reference to Fig. 1A and Fig. 4, the method according to the invention comprises an assessment step (F1) wherein a plurality of tests are performed to assess the user's visual abilities.

The assessment step (F1) comprises five tests (T, T1, T2, T3, T4), which will be described in detail hereinafter.

All tests (T, T1, T2, T3, T4) are executed through the visor (2), i.e., they are executed by having the user use the visor (2) and are managed by the viewing software (12) of the control unit (1) that projects figures, signs, and symbols on the display (200) of the visor (2).

With reference to Figs. 4, 5, 5A, 6, and 6A, the assessment step (F1) provides for executing a first test (T) to assess a monocular visual acuity in the right eye and in the left eye of a user to obtain a right visual acuity index (QD) and a left visual acuity index (QS).

The first test (T) provides for executing a right subtest and a left subtest, each one being suitable for assessing the visual acuity of the user's right eye and left eye.

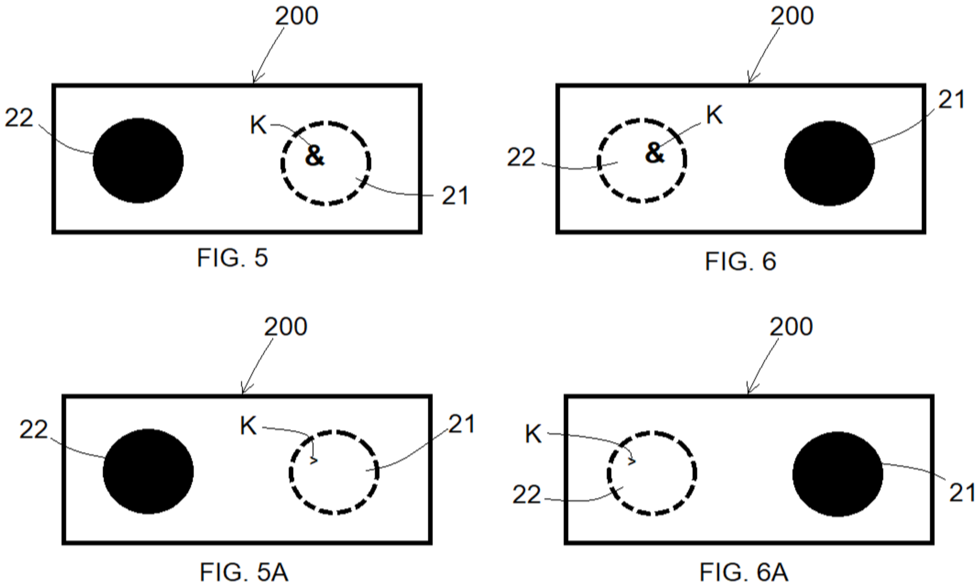

With reference to Figs. 4, 5, and 5A, the right subtest involves:

- covering the left eyepiece (22) of the visor (2) by means of covering means;
- projecting a sequence of symbols (K) with decreasing dimensions on the display (200);
- detecting the smallest symbol (K) the user can see and recognize with his/her right eye;
- defining the right visual acuity index (QD) based on the smallest symbol (K) that the user was able to see and recognize.

With reference to Figs. 4, 6, and 6A, the left subtest provides for:

- covering the right eyepiece (21) of the visor (2) by means of covering means and uncovering the left eyepiece (22);
- projecting a sequence of symbols (K) with decreasing dimensions on the display (200);
- detecting the smallest symbol (K) the user can see and recognize with his/her left eye;
- defining the left visual acuity index (QS) based on the smallest symbol (K) that the user was able to see and recognize.

In particular, the projection of symbols (K) with decreasing dimensions is performed on a scale of thirteen levels with values ranging from 1/50 to 10/10, as shown in Table 1 below:

Table 1

| LEVEL | Visual acuity index |
|-------|---------------------|
| Level 1 | 1/50 |
| Level 2 | 1/30 |
| Level 3 | 1/20 |
| Level 4 | 1/10 |
| Level 5 | 2/10 |
| Level 6 | 3/10 |
| Level 7 | 4/10 |
| Level 8 | 5/10 |
| Level 9 | 6/10 |
| Level 10 | 7/10 |
| Level 11 | 8/10 |
| Level 12 | 9/10 |
| Level 13 | 10/10 |

In level 1, which corresponds to 1/50 visual acuity level, the size of the symbol (K) reproduced on the display is maximum, whereas in level 13, which corresponds to 10/10 visual acuity level, the size of the symbol reproduced on the display is minimum.

Based on the level at which the user can still see the symbol (K) reproduced on the display (200), the level of right or left monocular visual acuity is defined.

Every time the user detects a symbol (K) on the display (200), he/she says aloud or types the detected symbol (K) on a keyboard. Therefore, the user's input (IN) can be either audible or mechanical, by pressing a specific button on a keyboard.

The user interface (4) may comprise a microphone that detects the user's voice indicating the detected symbol, or a keyboard consisting of a plurality of keys, each one being associated with a sign or symbol to be identified. It should be noted that the symbol spoken aloud by the user can be detected by the examiner, who then enters the input (IN) directly into a computer.

The calculation means (14) of the control unit (1) receive the input (IN) from the user interface (4) and compare the input (IN) with the sign/symbol (K) that is actually reproduced on the display (200) in order to verify whether the input (IN) and the sign/symbol (K) match or not.

Based on the level at which the user makes a mistake or fails to identify the symbol, the calculation means (14) generate a right (QD) or left (QS) visual acuity index that coincides with the monocular visual acuity level at which the user was able to identify the sign/symbol (K).

Once the first test (T) has been performed, a comparison step (W) is executed wherein a comparator (not shown in the attached figures) of the control unit (1) compares the right visual acuity index (QD) and the left visual acuity index (QS) in order to identify dominance information (Q) indicating which of the two eyes is the dominant eye and which is the non-dominant eye. Such dominance information (Q) on the dominant/non-dominant eye is stored in the memory (10).

More precisely, the comparator is configured to compare the right visual acuity index (QD) and the left visual acuity index (QS) to identify which of the two indices is greater and, based on this, define the dominance information (Q) on the dominant/non-dominant eye.

If the right (QD) and left (QS) visual acuity indices are equal, then the comparator randomly decides which of the two eyes is the dominant eye or the non-dominant eye, or such a decision can also be made by the examiner or doctor examining the patient.

Based on the comparison, the following is obtained:

| Comparison result | Dominance information (Q) |
|-------------------|---------------------------|
| QD>QS | Dominant right eye and non-dominant left eye |
| QS>QD | Dominant left eye and non-dominant right eye |

(continued)

| Comparison result | Dominance information (Q) |
|---|---|
| QS=QD | Dominant eye chosen by the comparator or examiner/doctor |

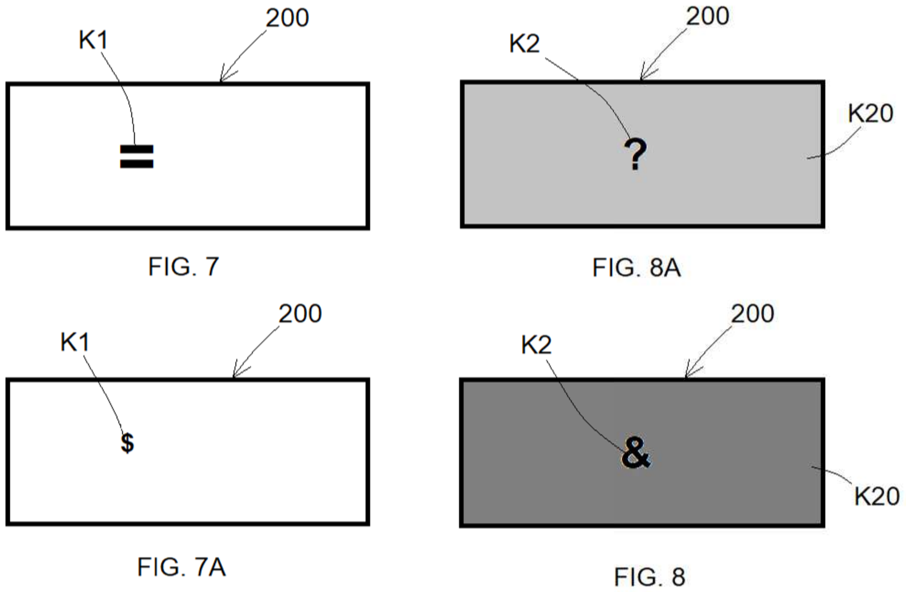

With reference to Figs. 4, 7, 7A, the assessment step (F1) provides for executing a second test (T1) to assess a binocular visual acuity, i.e., with both eyes open simultaneously, in order to obtain a binocular visual acuity index (Q1) and store such a binocular visual acuity index (Q1) in the memory (10) of the control unit (1).

In particular, said second test (T1) provides for:

- uncovering both eyepieces (21, 22) of the visor (2);
- projecting a sequence of symbols (K1) with decreasing dimensions on the display (200);
- detecting the smallest symbol (K1) the user can see and recognize with both eyes;
- defining the binocular visual acuity index (Q1) based on the symbol (K1) the user was able to see and recognize.

The symbols (K1) with decreasing dimensions are projected on a scale of thirteen levels, as the one described above and shown in Table 1 with reference to the first test (T).

Every time the user detects a symbol (K1) on the display (200), he/she says aloud or types the detected symbol (K1) on a keyboard. Therefore, the user's input (IN) can be either audible or mechanical by pressing a specific button on a keyboard.

The calculation means (14) of the control unit (1) therefore receive the input (IN) from the user interface (4) and compare the input (IN) with the sign/symbol (K1) actually reproduced on the display (200) in order to verify whether the input (IN) and the sign/symbol (K1) match or not.

Based on the level at which the user makes a mistake or fails to identify the symbol, the assessment means (14) generate a binocular visual acuity index (Q1) corresponding to the last binocular visual acuity level at which the user was able to identify the sign/symbol (K1). Such a binocular visual acuity index (Q1) is then stored in the memory (10) of the control unit (1).

With reference to Figs. 4, 8, and 8A, the assessment step (F1) further provides for executing a third test (T2) to assess the binocular contrast sensitivity in order to obtain a contrast sensitivity index (Q2) and store such a contrast sensitivity index (Q2) in the memory (10) of the control unit (1).

The third test (T2), which is performed with both eyes open, provides for:

- projecting a sequence of images on the display (200), each image comprising one or more symbols (K2) and a background (K20), gradually increasing the contrast level between the symbol (K2) and the background (K20);
- detecting the contrast level at which the user can distinguish the symbol (K2) from the background (K20);
- defining the contrast sensitivity index (Q2) based on the contrast level identified previously.

Practically, the third test (T2) provides for reproducing eight image levels with increasing contrast percentages on the display (200) that are gradually easier to perceive, following the sequence shown in Table 2 below:

Table 2

| Quality level | Contrast percentage |
|---|---|
| Level 1 | 0.8% |
| Level 2 | 1.5% |
| Level 3 | 2.9% |
| Level 4 | 5.5% |
| Level 5 | 10.5% |
| Level 6 | 19.9% |
| Level 7 | 37.8% |
| Level 8 | 72% |

By way of example, Fig. 8 shows a contrast of 1.5% (level 2), whereas Fig. 8A shows a contrast of 72% (level 8). Comparing Figs. 8 and 8A, it is evident that the higher the contrast, the easier it is to distinguish the symbol (K2) from the background (K20).

The level at which the user can perceive the symbol (K2) in binocular vision within a preset time of 10 seconds defines the individual contrast sensitivity level.

Every time the user identifies the symbol (K2) on the display (200) against the background (K20), the user says aloud or types on the keyboard the detected symbol (K2) and the user interface (4) (microphone or keyboard) sends the user's voice or mechanical input (IN).

When the symbol (K2) and the input (IN) match, the calculation means (14) generate the contrast sensitivity index (Q2) that coincides with the contrast level at which the user was able to distinguish the symbol (K2) from the background (K20). The contrast sensitivity index (Q2) is then stored in the memory (10).

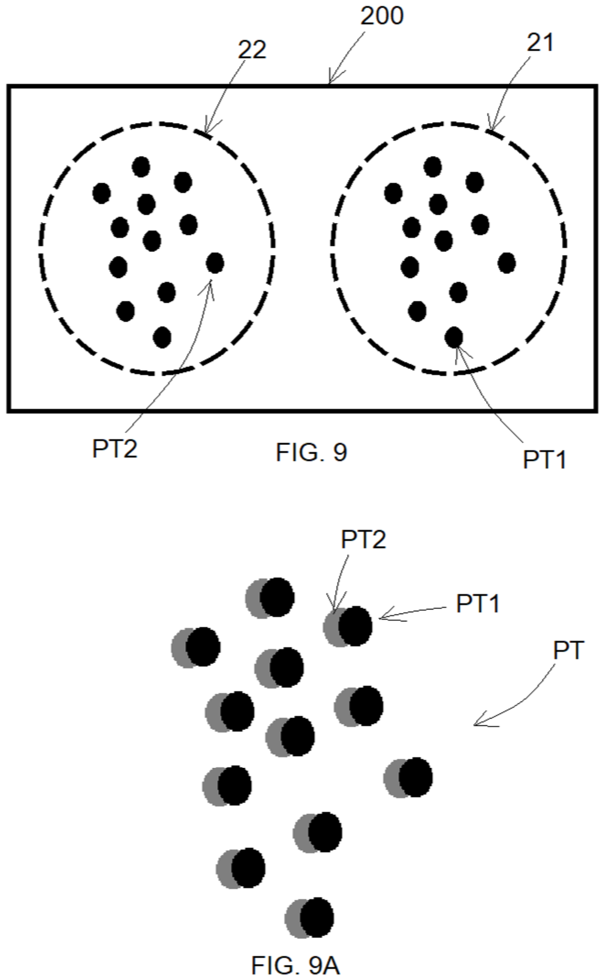

With reference to Figs. 4, 9, 9A, and 9B, the assessment step (F1) provides for executing a fourth test (T3) to assess the stereoscopic level in order to obtain a stereoscopic level index (Q3) and store such a stereoscopic level index (Q3) in the memory (10) of the control unit (1).

The fourth test (T3) is performed in binocular vision and provides for:

- projecting a sequence of images reproducing a set of induced stereograms (K31, K32) with decreasing stereoscopic perception difficulty on the display (200);
- detecting which image the user is able to perceive as a relief and three-dimensional image;
- defining the stereoscopic level index (Q3) based on the image in which the user was able to perceive a three-dimensional image.

Fig. 9 illustrates the example of a right pattern (PT1) and a left pattern (PT2) projected on the display (200) at the right eyepiece (21) and the left eyepiece (22), respectively. Each pattern is given by a plurality of luminous points. The two patterns (PT1, PT2) are identical to each other. The right pattern (PT1) is angularly offset in horizontal direction with respect to the left pattern (PT2). The offset is measured as an offset angle with respect to the center of the visor eyepiece. The offset angle is measured in seconds.

With reference to Fig. 9A, given that the user's eyes are resting on the eyepieces (21, 22) of the visor (and therefore they are close to the display (200)), the two patterns (PT1, PT2) are perceived as two superimposed images that constitute an induced stereogram (PT). The fact that the two patterns are offset and not perfectly superimposed creates a three-dimensional effect in the induced stereogram (PT).

The smaller the offset between the two patterns (PT1, PT2) that induces an impression of relief in the individually perceived stereogram (PT), the higher the stereoscopy level of the user.

The fourth test (T3) is executed by the control unit (1) using a program characterized by a seven-level scale in which the angular offset between the two patterns (PT1, PT2) is measured in seconds and increases progressively in order to gradually facilitate the perception of relief.

Preferably, the following level scale shown in Table 3 is used:

Table 3

| Level | Angular distance at which a sense of depth is perceived |
|---|---|
| level 1 | 10" |
| level 2 | 20" |
| level 3 | 40" |
| level 4 | 80" |
| level 5 | 120" |
| level 6 | 160" |
| level 7 | 320" |

If a user is able to detect a perception of relief in the early levels, it means that the user has a good stereoscopic ability; whereas, if the user is only able to detect a perception of relief in the last levels, it means that the user has a reduced stereoscopic ability.

When the user detects a three-dimensional image and perceives a three-dimensional symbol generated by the induced stereogram (PT), the user says the characteristics of the symbol aloud or presses a specific button on the keyboard. The user's voice or the pressing of the button represents the input (IN) generated by the user via the user interface (4).

The calculation means (14) of the control unit (1) receive the input (IN) and generate the stereoscopic level index (Q3) coinciding with the level of the fourth test (T3) in which the user was able to detect a three-dimensional image.

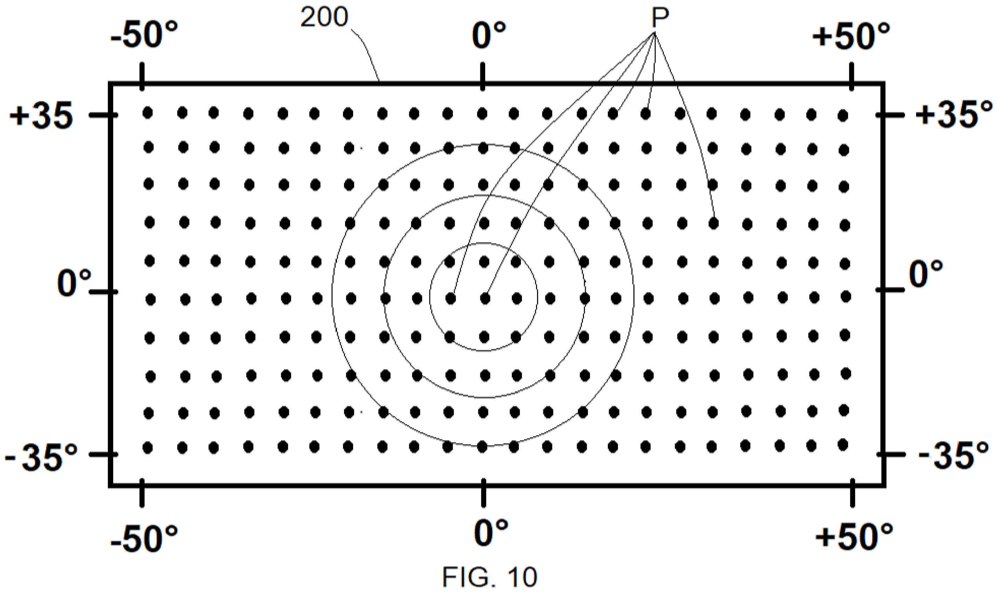

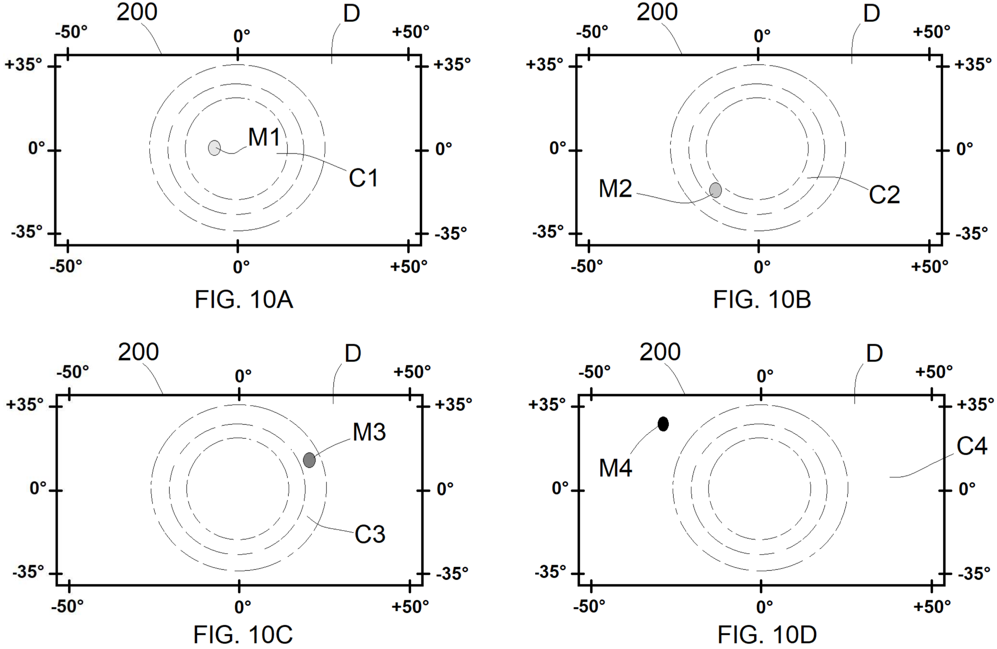

With reference to Figs. 4, 10, 10A, 10B, 10C, and 10D, the assessment step (F1) also provides for executing a fifth test (T4) to assess the binocular field of view, so as to obtain a plurality of luminous perception indices (L1, L2, L3, L4, ....), each of them being associated with a specific area of the user's binocular field of view and store said luminous perception indices (L1, L2, L3, L4, ....) in the memory (10) of the control unit (1).

The fifth test (T4) provides for executing a plurality of tests by displaying a background (D) and luminance targets (M1, M2, M3, M4) on the display (200) of the visor (2) in the form of luminous dots disposed in specific positions that appear in the background one at a time and the user reports their perception via the user interface (4).

The positions of the luminance targets (M1, M2, M3, M4) correspond to analysis points (P) of the user's field of view.

Advantageously, the display screen is divided into analysis points (P) distributed along rows and columns and positioned within 100° on a horizontal plane and 70° on a vertical plane.

For each test, the N luminance targets (M1, M2, M3, M4) appear progressively, one by one, on the display (200), and the patient reports their vision using the user interface (4). For each test, the brightness value of the luminance targets (M1, M2, M3, M4) increases as they move away from the center of the display (200) towards the periphery.

More precisely, the display (200) is divided into four concentric zones, namely a first central zone (C1), a second zone (C2), a third zone (C3), and a fourth peripheral zone (C4). In Figures 10, 10A, 10B, and 10C, the four zones (C1, C2, C3, C4) are separated by imaginary circular dotted lines.

The luminance targets (M1) that appear in the first central zone (C1) have a first brightness, the luminance targets (M2) that appear on the second zone (C2) have a second brightness greater than the first brightness, the targets (M3) that appear on the third zone (C3) have a third brightness greater than the second brightness, whereas the targets (M4) that appear on the fourth zone (C4) have a fourth brightness greater than the third brightness.

In a first test, the luminance targets (M1, M2, M3, M4) are presented one after the other in random order at all analysis points (P), and the user reports seeing them.

In the tests following the first initial test, only the luminance targets not seen in the previous test appear progressively, one by one, on the display (200) at the same brightness as in the previous test or at a higher brightness than in the previous test, and the user reports seeing them.

The user can use the user interface (4) to report seeing them, such as a button that is pressed by the user each time a target is perceived.

In a second test, the targets not seen by the user are repeated with the same brightness as in the first test.

In a third test, the targets from the second test that were not seen by the user are presented again with a higher brightness than in the second test.

In a fourth test, the targets from the third test that were not seen by the user are presented again with the same brightness as in the third test.

Subsequently, twelve additional tests are executed for a total of sixteen tests with the same criterion used for the previous tests. More precisely, every two tests, the brightness of the unseen targets is increased.

In particular, the fifth test (T4) consists of sixteen tests grouped two by two in eight brightness levels according to the scheme shown in Table 4:

Table 4

|  |  | Luminance target (M1) projected in first zone (C1) | Luminance target (M2) projected in second zone (C2) | Luminance target (M3) projected in third zone (C3) | Luminance target (M4) projected in fourth zone (C4) |
|---|---|---|---|---|---|
| Test 1 | Brightness level 1 | 1.4 lux | 1.5 lux | 1.6 lux | 1.7 lux |
| Test 2 |  |  |  |  |  |
| Test 3 | Brightness level 2 | 2.5 lux | 2.7 lux | 2.9 lux | 3.1 lux |
| Test 4 |  |  |  |  |  |
| Test 5 | Brightness level 3 | 4.5 lux | 4.8 lux | 5.1 lux | 5.4 lux |
| Test 6 |  |  |  |  |  |
| Test 7 | Brightness level 4 | 8.2 lux | 8.7 lux | 9.2 lux | 9.7 lux |
| Test 8 |  |  |  |  |  |
| Test 9 | Brightness level 5 | 14.8 lux | 15.7 lux | 16.6 lux | 17.5 lux |
| Test 10 |  |  |  |  |  |

(continued)

| | | Luminance target (M1) projected in first zone (C1) | Luminance target (M2) projected in second zone (C2) | Luminance target (M3) projected in third zone (C3) | Luminance target (M4) projected in fourth zone (C4) |
|---|---|---|---|---|---|
| Test 11 | Brightness level 6 | 26.5 lux | 28.1 lux | 29.7 lux | 31.3 lux |
| Test 12 | | | | | |
| Test 13 | Brightness level 7 | 47.6 lux | 50.5 lux | 53.4 lux | 56.3 lux |
| Test 14 | | | | | |
| Test 15 | Brightness level 8 | 80 lux | 84.8 lux | 89.6 lux | 94.5 lux |
| Test 16 | | | | | |

Once the fifth test (T4) has been completed, the corresponding luminous perception index (L1, L2, L3, L4, etc.) is calculated for each point examined.

The luminous perception index is calculated directly by the control unit (1) using the calculation means (14) that take into account the targets seen in the various tests of the fifth test (T4). The luminous perception indices (L1, L2, L3, L4, etc.) are then stored in the memory (10).

The luminous perception index (L1, L2, L3, L4....) can be a value indicating at what brightness level the target was seen at the corresponding analysis point (P).

Once the assessment step (F1) is complete, the following data will be stored in the memory (10) of the control unit (1):

- dominance information (Q) indicating which of the two eyes is the dominant eye and which is the non-dominant eye;
- binocular visual acuity index (Q1) in the form of a value chosen from: 1/50, 1/30, 1/20, 1/10, 2/10, 3/10, 4/10, 5/10, 6/10, 7/10, 8/10, 9/10, 10/10;
- contrast sensitivity index (Q2) in the form of a level number: Level 1, Level 2, Level 3, Level 4, Level 5, Level 6, Level 7, Level 8;
- stereoscopic level index (Q3) in the form of a level number: Level 1, Level 2, Level 1, Level 2, Level 3, Level 4, Level 5, Level 6, Level 7,
- a plurality of luminous perception indices (L1, L2, L3, L4, ....), each one being associated with a specific analysis point of the user's binocular field of view corresponding to the brightness level of the scale at which the luminance target (M1, M2, M3, M4) was seen at that specific analysis point.

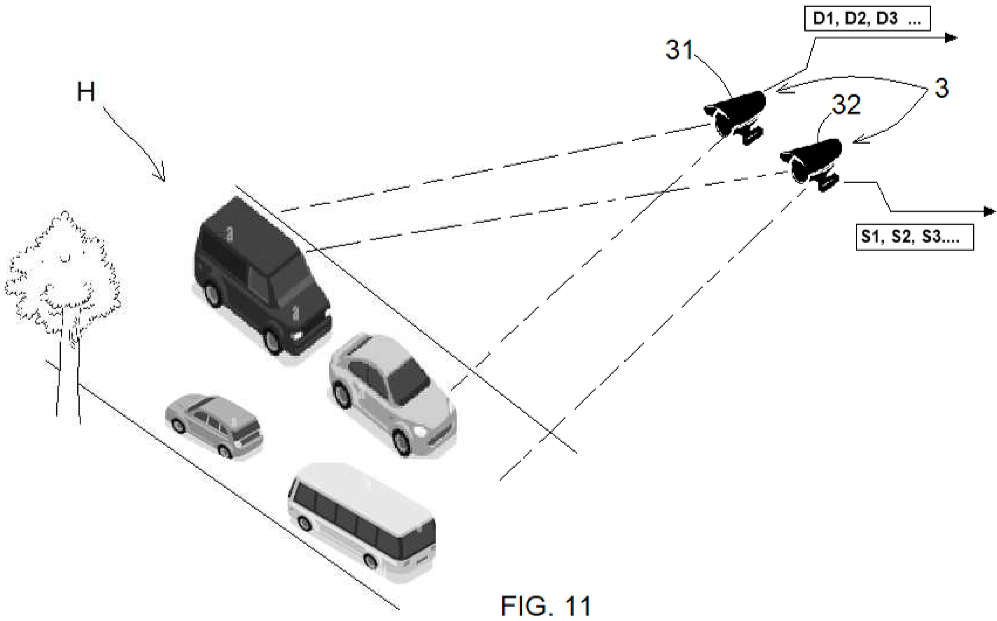

With reference to Figs. 1A and 11, the method according to the invention also comprises an acquisition step (F2) that provides for acquiring a plurality of sequential images of the same scene (H) from two different positions by means of said image acquisition means (3), so as to have a plurality of right images (D1, D2, D3.....) and a plurality of left images (S1, S2, S3....). The image acquisition means (3) comprise two cameras (31, 32).

For illustrative purposes, Fig. 11 schematically shows the acquisition of images of a scene (H) relative to a road with vehicle traffic.

Once the assessment step (F1) and the acquisition step (F2) are completed, the method comprises a post-processing step (F3) of the images (D1, D2, D3....., S1. S2. S3), wherein the images (D1, D2, D3....., S1, S2, S3) are processed taking into account the dominance information (Q) and the indices (Q, Q1, Q2, Q3; L1, L2, L3, L4....) stored in the memory (10) and obtained during the tests (T, T1, T2, T3, T4).

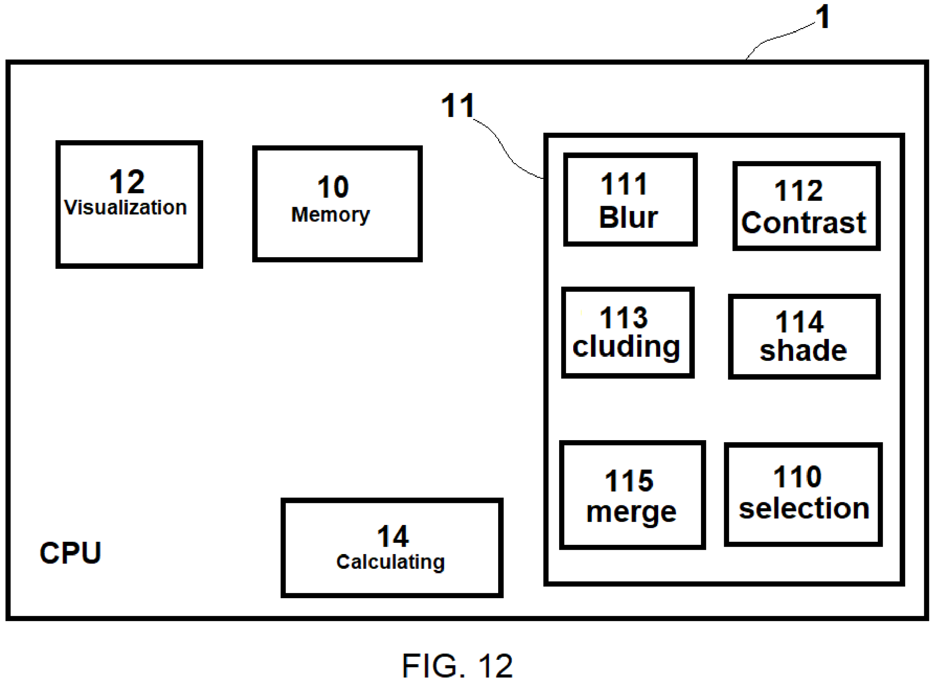

With reference to Fig. 12, the post-processing step (F3) is executed by the image processing software (11) that comprises a blur program (111), a contrast adjustment program (112), a selection program (110), a cloud program (113), a merge program (115), and a shade program (114).

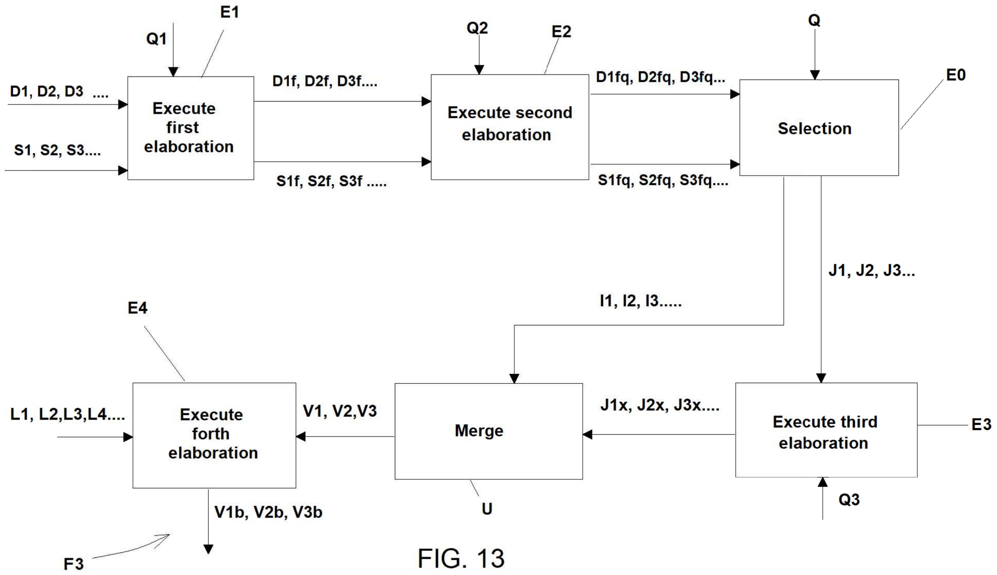

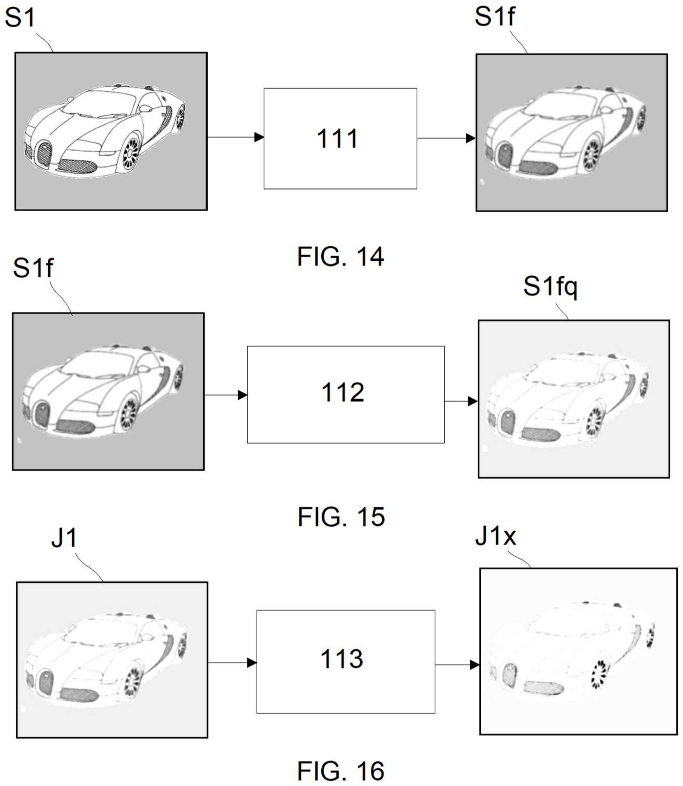

With reference to Figs. 13 and 14, the post-processing step (F3) firstly provides for performing a first processing (E1) of the right images (D1, D2, D3....) and of the left images (S1, S2, S3....) with the blur program (111) of the image processing software (11), which is configured in such a way as to generate a blur on the right and left images (D1, D2, D3, S1, S2, S3) in proportion to the binocular visual acuity index (Q1), so as to obtain blurred right images (D1f, D2f, D3f) and blurred left images (S1f, S2f, S3f).

The blur program (111) comprises a blur shader, which is a public domain mini-program suitable for receiving a scalar blur parameter as input and uniformly blurring the right and left images (D1, D2, D3..., S1, S2, S3...) based on the scalar blur parameter.

By way of example, a Gaussian blur shader can be used as blur shader.

The scalar parameter must be a value ranging from 0 (no blur) to a maximum value.

In this regard, it is necessary to convert the binocular visual acuity index (Q1) into said blur scalar parameter (PSS) to be sent to the shader.

By way of example, it is possible to use an inverse relationship that links the binocular visual acuity index (Q1) and the blur scalar parameter (PSS) as in the following equation:

$$PSS = 1 - \left(\frac{Q1}{\max(Q1)}\right)$$

where:

- Q1 is the binocular visual acuity index (Q1) calculated during the second test (T1);
- max(Q1) is the maximum value of the visual acuity scale equal to 10/10; and
- PSS is the scalar blur parameter.

Therefore, the scalar blur parameter (PSS) for a binocular visual acuity index (Q1) of 1/50 will be close to 1, which is the maximum blur level, whereas for a binocular visual acuity value (Q1) of 10/10 it will be equal to 0 (no blur).

With reference to Figs. 13 and 15, successively, the post-processing step (F3) provides for executing a second processing (E2) of the blurred right images (D1f, D2f, D3f) and of the blurred left images (S1f, S2f, S3f) with the contrast adjustment program (112) of the image processing software (11) that adjusts the contrast of the blurred images (D1f, D2f, D3f.... ; S1f, S2f, S3f.....) in proportion to the contrast sensitivity index (Q2), so as to obtain contrast-modified blurred left images (S1fq, S2fq, S3fq......) and contrast-modified blurred right images (D1fq, D2fq, D3fq....;).

Preferably, the contrast adjustment program (112) is an alpha shader, which is known to an expert of the field and of public domain, which receives a contrast adjustment parameter as input and modifies the contrast of the blurred left and right images (D1f, D2f, D3f.... ; S1f, S2f, S3f....) based on the scalar contrast adjustment parameter.

The applicant has created an algorithm that correlates the level of the contrast sensitivity index (Q2) obtained from the third test (T2) with the corresponding contrast adjustment parameter to be input to the alpha shader.

In this regard, a first look-up table is stored in the memory (10) of the control unit (1), wherein each level of the contrast sensitivity index (Q2) is associated with the corresponding contrast adjustment parameter. The first look-up table is shown in Table 5.

Table 5

| Contrast sensitivity index level (Q2) | Contrast adjustment parameter |
| --- | --- |
| Level 1 | 0% |
| Level 2 | 1.5% |
| Level 3 | 2.9% |
| Level 4 | 5.5% |
| Level 5 | 10.5% |
| Level 6 | 19.9% |
| Level 7 | 37.8% |
| Level 8 | 72% |

If the user has an optimal contrast level (level 1), the right and left blurred images (D1f, D2f, D3f.... ; S1f, S2f, S3f....) will not be modified (filtered) by the alpha shader; whereas if the user has a contrast level greater than 1, then the images will be modified (filtered) progressively as the level increases.

With reference to Fig. 13, the post-processing step (F3) provides for executing a selection step (E0) with the selection program (110) wherein, based on the dominance information (Q) relating to the dominant eye and the non-dominant eye, the contrast-modified blurred images (D1fq, D2fq, D3fq....; S1fq, S2fq, S3fq......) associated with the dominant eye are selected as dominant images (I1, I2, I3.......), whereas the contrast-modified blurred images (D1fq, D2fq, D3fq....; S1fq, S2fq, S3fq......) associated with the non-dominant eye are selected as non-dominant images (J1, J2, J3 .....). By way of example, let's say that, based on the dominance information (Q), the user has the right eye as the dominant eye and the left eye as the non-dominant eye. In such a case, the contrast-modified blurred right images (D1fq, D2fq, D3fq....) are classified as dominant images (I1, I2, I3....), whereas the contrast-modified blurred left images (S1fq, S2fq, S3fq....) are classified as non-dominant images (J1, J2, J3 ....).

With reference to Figs. 13 and 16, once the dominant images (I1, I2, I3) and the non-dominant images (J1, J2, J3) have been selected, a third processing (E3) is executed only on the non-dominant images (J1, J2, J3) using the cloud program (113) which clouds the non-dominant images (J1, J2, J3 .....) in proportion to the stereoscopic level index (Q3) in order to obtain clouded non-dominant images (J1x, J2x, J3x....).

Also in such a case, the cloud program (113) may comprise a public domain shader or a program specifically designed to create a cloud that is directly proportional to the stereoscopic level index (Q3).

Otherwise said, the higher (worse) the user's stereoscopy level that coincides with the stereoscopic level index (Q3), the higher the cloud of the non-dominant images (J1, J2, J3 .....) by the cloud program (113) will be.

Also in such a case, the applicant has created an algorithm that relates the stereoscopic level index (Q3) to a corresponding cloud parameter to be input to the cloud program (113).

In this regard, a second look-up table is stored in the memory (10) of the control unit (1), in which each stereoscopic level index (Q3) is associated with the corresponding cloud parameter. The second look-up table is shown in Table 6.

Table 6

| Level 1 | 0% |
|---|---|
| Level 2 | 2% |
| Level 3 | 4% |
| Level 4 | 8% |
| Level 5 | 12% |
| Level 6 | 16% |
| Level 7 | 32% |

If the fourth test (T3) has shown that the user has an optimal stereoscopic level (level 1), then the non-dominant images (J1, J2, J3, etc.) will not be aberrated.

Conversely, if the fifth test (T4) has shown that the user has a very poor stereoscopic level (level 7), then the non-dominant images (J1, J2, J3) will be massively aberrated with 32% cloud.

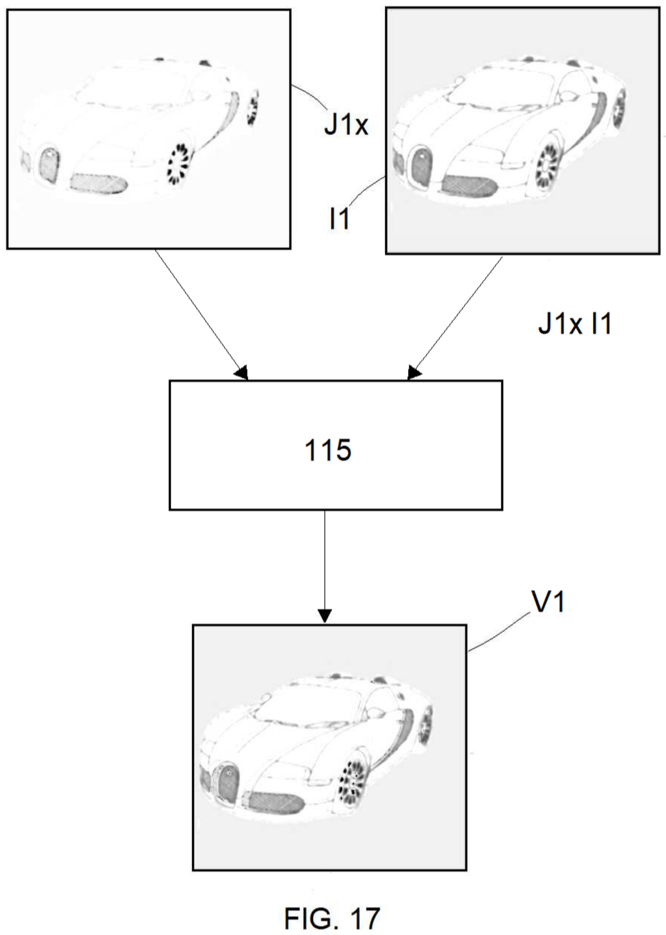

With reference to Figs. 13 and 17, the post-processing step (F3) provides for executing a merge step (U) using the merge program (115) of the image processing software (11), in which the dominant images (I1, I2, I3.....) and the clouded non-dominant images (J1x, J2x, J3x) are merged/combined, two by two, in such a way as to obtain a plurality of binocular images (V1, V2, V3 .....).

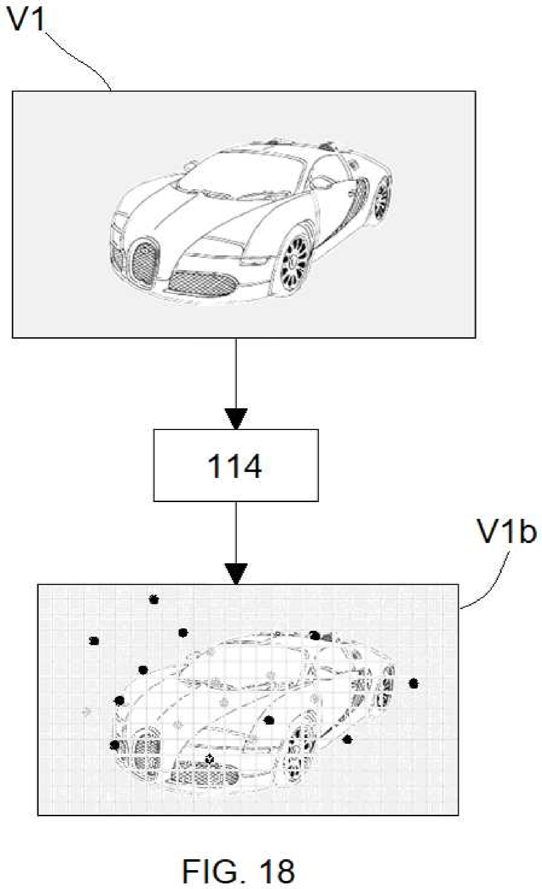

With reference to Figs. 13 and 18, the post-processing step (F3) further provides for executing a fourth processing (E4) of the binocular images (V1, V2, V3), using the shade program (114) of the image processing software (11). The shade program (114) is configured in such a way as to:

- divide each binocular image (V1, V2, V3) into a grid composed of a number of circular areas equal to the number of the analysis points (P) of the binocular field of view tested in the fifth test (T4); wherein each circular area of the grid is associated with an analysis point (P) assessed in the fifth test (T4);
- apply a shade to each circular area of each binocular image (V1, V2, V3....), in proportion to the luminous perception index (L1, L2, L3, L4....) of the corresponding analysis point (P) of the binocular visual field tested in the fifth test (T4), in such a way as to obtain a plurality of aberrated binocular images (V1b, V2b, V3b....).

To calculate the amount of shade to be applied to each circular area of the binocular images (V1, V2, V3...), also in this case, the applicant has devised a relationship linking the luminous perception index (L1, L2, L3, L4.....) with the shade level to be applied to the circular area corresponding to the analysis point (P).

In this regard, a third look-us table is stored in the memory (10) of the control unit (1), in which each luminous perception index (L1, L2, L3, L4.....) is associated with a shade level. The third look-up table is shown in Table 6.

Table 6

| Luminous perception index | Shade level |
|---|---|
| Perception of corresponding target at brightness level 1 | 0% |
| Perception of corresponding target at brightness level 2 | 2.5% |

(continued)

| Luminous perception index | Shade level |
|---|---|
| Perception of corresponding target at brightness level 3 | 4.5% |
| Perception of corresponding target at brightness level 4 | 8.2% |
| Perception of corresponding target at brightness level 5 | 14.8% |
| Perception of corresponding target at brightness level 6 | 26.5% |
| Perception of corresponding target at brightness level 7 | 47.6% |
| Perception of corresponding target at brightness level 8 | 80% |

The shade is applied to each circular area of the grid using a Gaussian filter centered in the center point of the circular area.

The application of the shade for each zone through circular areas of defined radius can be performed using a commercially available software or through a specially developed procedure. By way of example, the commercially available software program known as "coords in scotoma" can be used.

The edges of the circular areas are shaded using a Gaussian shade program, such as the commercially available software program known as "mask=cv2".

Finally, with reference to Fig. 13, the method provides for performing a video playback step (F4) wherein the aberrated binocular images (V1b, V2b, V3b) are reproduced on the display (200) of the visor (2) in sequence in such a way as to obtain a video (V) simulating the user's vision.

Following the above description, the advantages of the present invention are evident. In fact, the new method according to the invention can have multiple potential applications in different fields.

For illustrative purposes, such a method can be used in all cases where it is necessary to assess the impact of a visual impairment on the total disability of the patient (pensions and subsidies issued from National Social Security Institutes and assessments executed by Workers' Compensation Authorities).

The proposed method makes it possible to highlight the actual ability of visually impaired patients to carry out their daily activities, interfacing their actual vision with scenes that reproduce the environment in which they usually move. With such a method, it is possible to propose a virtually unlimited number of real-life situations through 3D visors.

The method can also be used to recreate traffic accident scenes from the perspective perceived by drivers at the time of the event. Thanks to this method, it is possible to recreate the accident scene as perceived by the protagonist's eyes, and modulate it by reproducing any visual impairments that the protagonist may have had at the time of the accident. It is also possible to reproduce the accident scene dynamically (modulating the speed of the vehicles as deduced from the measurements taken by the police) and modify the scene according to the particular environmental conditions (brightness, rain, fog, glare of various kinds, etc.) present at the time of the accident.

The method can also be advantageously applied in the field of insurance. By way of example, when taking out a car insurance policy, the various companies can modulate the cost of the policies based on the analysis of the videos that reproduce the actual individual vision by comparing it with the risk rate linked to individual visual abilities.

Furthermore, the method can be used in the field of occupational medicine, where it is possible to interface the characteristic scenes of a user's work environment with the user's actual vision in order to optimize the selection at the time of hiring, optimize the distribution of internal company resources, and reduce the risk rate for categories of workers who perform specific roles.

The method according to the invention can be applied in the field of sports medicine, wherein it is necessary to assess how an athlete's visual impairment can affect his/her performance.

Finally, an additional advantage of the present method is that the various tests are not executing in an environment (that could be affected by external agents), but they are performed using a special visor that allows for realizing highly reproducible test conditions independently of the external environmental variables that can significantly alter the results.

**Claims**

1. Method for generating and displaying a video that simulates a user's vision; said method being implemented by a system (100) comprising:

   - a visor (2) suitable for being used by a user; wherein said visor (2) comprises a display (200) for displaying images and videos;
- image acquisition means (3) set up and configured to acquire images (D1, D2, D3..... ; S1, S2, S3) of a scene (H) from two different positions, so as to simulate the vision of a right eye and a left eye;
- a control unit (1) comprising an image processing software (11) and a memory (10); wherein said image acquisition means (3) and said visor (2) are operatively connected to the control unit (1);

wherein said method comprises the following steps:

- an assessment step (F1) wherein a plurality of tests (T, T1, T2, T3, T4) are performed on the user to assess the user's visual abilities and information and indices (Q, Q1, Q2, Q3; L1, L2, L3, L4....) on the user's visual abilities are collected and stored in the memory (10) of the control unit (1);
- an acquisition step (F2) using said image acquisition means (3), wherein a plurality of sequential images of the same scene (H) are acquired from said two different positions, so as to have a plurality of right images (D1, D2, D3.....) and a plurality of left images (S1, S2, S3....) that simulate the vision of a right eye and a left eye;
- a post-processing step (F3) wherein said right images (D1, D2, D3.....) and said left images (S1, S2, S3....) are processed by an image processing software (11) of the control unit, based on said information and indices (Q, Q1, Q2, Q3, L1, L2, L3, L4....) detected in said assessment step (F1), so as to obtain aberrated binocular images (V1b, V2b, V3b), and
- a video playback step (F4) wherein said aberrated binocular images (V1b, V2b, V3b) are displayed on the display (200) of the visor (2), by means of a visualization software (12) of the control unit (1), so as to playback said video (V) simulating the vision of a user.

2. The method according to claim 1, wherein said assessment step (F1) provides for having the user use the visor (2); wherein said tests (T, T1, T2, T3, T4) are managed by a visualization software (12) of the control unit (1) which projects figures, signs, symbols on the display (200) of the visor (2).

3. The method according to claim 1 or 2, wherein the system (100) used to implement the method comprises a user interface (4) comprising means configured to detect the user's input (IN) during the performance of the tests (T, T1, T2, T3, T4); wherein during said assessment step (F1), said control unit (1) receives said inputs (IN) to calculate the information and indices (Q, Q1, Q2, Q3, L1, L2, L3, L4....) relating to each test (T, T1, T2, T3, T4) performed by the user by means of calculation means (14).

4. The method according to claim 1 or 2 or 3, wherein said assessment step (F1) comprises the following operational steps:

- performing a first test (T) to assess a monocular visual acuity in the user's right eye and left eye in order to obtain a right visual acuity index (QD) and a left visual acuity index (QS);
- comparing (W) the right visual acuity index (QD) and the left visual acuity index (QS) in order to identify dominance information (Q) indicating which of the user's two eyes is the dominant eye and which is the non-dominant eye;
- performing a second test (T1) to assess a binocular visual acuity in both of the user's eyes in order to obtain a binocular visual acuity index (Q1).
- performing a third test (T2) to assess the contrast sensitivity in binocular vision in order to obtain a contrast sensitivity index (Q2);
- performing a fourth test (T3) to assess a stereoscopic level in order to obtain a stereoscopic level index (Q3); and
- performing a fifth test (T4) to assess the binocular visual field in order to obtain a plurality of luminous perception indices (L1, L2, L3, L4, ....), each one being associated with a specific analysis point (P) of the user's binocular field of view.

5. The method according to claim 4, wherein said post-processing step (F3) comprises the following operational steps:

- performing a first processing (E1) of the right images (D1, D2, D3....) and the left images (S1, S2, S3....) with a blur program (111) of the image processing software (11) which is configured in such a way as to generate blurring on the right and left images (D1, D2, D3, S1, S2, S3) in proportion to the binocular visual acuity index (Q1), so as to obtain blurred right images (D1f, D2f, D3f) and blurred left images (S1f, S2f, S3f);
- performing a second processing (E2) of the blurred right images (D1f, D2f, D3f) and blurred left images (S1f, S2f, S3f) with a contrast adjustment program (112) of the image processing software (11) that adjusts the contrast of the blurred images (D1f, D2f, D3f.... ; S1f, S2f, S3f.....) in proportion to the contrast sensitivity index (Q2), so as to obtain contrast-modified blurred left images (S1fq, S2fq, S3fq......) and contrast-modified blurred right images (D1fq, D2fq, D3fq....;);

- performing a selection step (E0) with a selection program (110) of the image processing software (11), wherein, based on the dominance information (Q) relating to the dominant eye and the non-dominant eye, the contrast-modified blurred images (D1fq, D2fq, D3fq....; S1fq, S2fq, S3fq......) associated with the dominant eye are selected as dominant images (I1, I2, I3.......), whereas the contrast-modified blurred images (D1fq, D2fq, D3fq....; S1fq, S2fq, S3fq......) associated with the non-dominant eye are selected as non-dominant images (J1, J2, J3 .....);

- performing a third processing (E3) only on the non-dominant images (J1, J2, J3....), using a cloud program (113) of the image processing software (11), which clouds the non-dominant images (J1, J2, J3 .....) proportionally to the stereoscopic level index (Q3), in order to obtain clouded non-dominant images (J1x, J2x, J3x....);

- performing a merge step (U) of the dominant images (I1, I2, I3...) and the clouded non-dominant images (J1x, J2x, J3x) in pairs using a merge program (115) of the image processing software (11) in order to obtain a plurality of binocular images (V1, V2, V3 .....).

- performing a fourth processing (E4) of the binocular images (V1, V2, V3), using a shade program (114) of the image processing software (11);

wherein said shade program (114) of the image processing software (11) is configured to:

- divide each binocular image (V1, V2, V3) into a grid composed of a number of circular areas equal to the number of analysis points (P) of the ocular field of view tested in the fifth test (T4); wherein each circular area of the grid is associated with an analysis point (P) assessed in the fifth test (T4);

- apply a shade to each circular area of each binocular image (V1, V2, V3....), in proportion to the luminous perception index (L1, L2, L3, L4....) of the corresponding analysis point (P) of the binocular field of view tested in the fifth test (T4), so as to obtain a plurality of aberrated binocular images (V1b, V2b, V3b....).

6. The method according to claim 4 or 5 when dependent on claim 2, wherein said visor (2) comprises a right eyepiece (21) against which the user's right eye is suitable for resting, and a left eyepiece (22) against which the user's left eye is suitable for resting; wherein said first test (T) provides for performing a right subtest and a left subtest for assessing the visual acuity of the right eye and the visual acuity of the left eye, respectively;

wherein said right subtest provides for:

- covering the left eyepiece (22) of the visor (2);
- projecting a sequence of symbols (K) of decreasing dimensions on the display (200);
- detecting the smallest symbol (K) that the user is able to see and recognize with the right eye;
- defining the right visual acuity index (QD) based on the smallest symbol (K) that the user was able to see and recognize;

wherein said left subtest comprises:

- covering the right eyepiece (21) of the visor (2) and uncovering the left eyepiece (22);
- projecting a sequence of symbols (K) of decreasing dimensions on the display (200);
- detecting the smallest symbol (K) the user can see and recognize with the left eye;
- defining the left visual acuity index (QS) based on the smallest symbol (K) that the user was able to see and recognize.

7. The method according to claim 4 or 5 when dependent on claim 2 or according to claim 6, wherein said second test (T1) provides for:

- uncovering both eyepieces (21, 22) of the visor (2);
- projecting a sequence of symbols (K1) of decreasing dimensions on the display (200);
- detecting the smallest symbol (K1) the user can see and recognize with both eyes;
- defining the binocular visual acuity index (Q1) based on the smallest symbol (K1) that the user was able to see and recognize.

8. The method according to claim 4 or 5 when dependent on claim 2 or according to claim 6 or 7, wherein said third test (T2) provides for:

- projecting a sequence of images on the display (200), each image comprising a symbol (K2) and a background (K20), gradually increasing the contrast level between the symbol (K2) and the background (K20);
- detecting at which contrast level the user is able to distinguish the symbol (K2) from the background (K20);
- defining the contrast sensitivity index (Q2) based on the contrast level identified previously.

9. The method according to claim 4 or 5 when dependent on claim 2 or according to claim 6 or 7 or 8, wherein said fifth test (T4) provides for:

- projecting a sequence of images reproducing a set of induced stereograms (K31, K32) with decreasing difficulty in terms of stereoscopic perception on the display (200);
- detecting which image the user is able to perceive as a relief and three-dimensional image;
- defining the stereoscopic level index (Q3) based on the image in which the user was able to detect a three-dimensional image.

10. The method according to claim 4 or 5 when dependent on claim 2 or according to claim 6 or 7 or 8 or 9, wherein said fifth test (T4) provides for executing a plurality of tests that involve displaying a background (D) and luminance targets (M1, M2, M3, M4) in the form of luminous dots disposed in specific positions that appear in the background (D), one at a time, and the user reports seeing them; wherein the positions in which the luminance targets (M1, M2, M3, M4) appear correspond to the analysis points (P) of the user's field of view;

wherein, in a first test, the luminance targets (M1, M2, M3, M4) are presented one after the other in random order at all the analysis points (P) of the visual field, and the user reports seeing them;
where, in the tests following the first test, only the luminance targets not seen in the previous test appear progressively, one by one, on the display (200) at the same brightness as the previous test or at a higher brightness than the previous test, and the user reports seeing them.

11. System (100) for implementing the method according to any of claims 1 to 10, wherein said system comprises:

- a visor (2) suitable for being used by a user; wherein said visor (2) comprises a display (200) for displaying images and videos;
- image acquisition means (3) set up and configured to acquire images (D1, D2, D3..... ; S1, S2, S3) of a scene (H) from two different positions, so as to simulate the vision of a right eye and a left eye;
- a control unit (1) comprising image processing software (11) and a memory (10); wherein said image acquisition means (3) and said visor (2) are operatively connected to the control unit (1);

wherein said image processing software (11) is configured to process said right images (D1, D2, D3.....) and said left images (S1, S2, S3....), based on the information and indices (Q, Q1, Q2, Q3, L1, L2, L3, L4....) detected in the assessment step (F1) of the method, so as to obtain the aberrated binocular images (V1b, V2b, V3b),
wherein said control unit (1) also comprises visualization software (12) configured to reproduce the aberrated binocular images (V1b, V2b, V3b) in sequence on the display (200) of the visor (2) in order to obtain the video (V) emulating the user's vision.

12. The system (100) according to claim 11, wherein said visualization software (12) is configured to manage the tests (T, T1, T2, T3, T4) of the assessment step (F1) of the method by projecting figures, signs, symbols on the display (200) of the visor (2).

13. The system (100) according to claim 11 or 12, comprising a user interface (4) comprising means configured to detect a user's input (IN) during the execution of the tests (T, T1, T2, T3, T4); wherein said control unit (1) comprises calculation means (14) configured to receive the inputs (IN) from the user interface (4) and calculate an assessment, information, or visual index relating to each test (T, T1, T2, T3, T4) performed by the user.

FIG. 1

Test → Valutation (F1) → Post-processing (F3) → Playback (F4) → V

H → Scene → Acquisition (F2)

D1, D2, D3
S1, S2, S3

Q, Q1, Q2, Q3, Q4

V1b, V2b, V3b

FIG. 1A

FIG. 2

EP 4 730 304 A1

FIG. 3

EP 4 730 304 A1

FIG. 4

EP 4 730 304 A1

FIG. 5

FIG. 6

FIG. 5A

FIG. 6A

200

K20

K2

FIG. 8A

200

K20

K2

FIG. 8

200

K1

FIG. 7

200

K1

FIG. 7A

FIG. 9

FIG. 9A

FIG. 10

EP 4 730 304 A1

FIG. 10A

FIG. 10B

FIG. 10C

FIG. 10D

FIG. 11

FIG. 12

CPU

1

11

12 Visualization

10 Memory

14 Calculating

111 Blur

112 Contrast

113 cluding

114 shade

115 merge

110 selection

FIG. 13

EP 4 730 304 A1

FIG. 14

FIG. 15

FIG. 16

28

J1x

I1

J1x I1

115

V1

FIG. 17

V1

114

V1b

FIG. 18

## EUROPEAN SEARCH REPORT

**Application Number**

EP 25 20 8757

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2019/160962 A1 (WERBLIN FRANK [US]) 22 August 2019 (2019-08-22) * paragraphs [0007] - [0051]; figures 1-15B * ----- | 1-13 | INV. G09B21/00 G06T5/00 G06T7/00 A61B3/00 |
| A | WO 2008/070683 A1 (FATEH SINA [US]) 12 June 2008 (2008-06-12) * paragraphs [0011] - [0024]; figures 1-10 * ----- | 1-13 | A61B3/02 A61B3/032 A61B3/08 |

TECHNICAL FIELDS
SEARCHED      (IPC)

G06T
A61B
G09B

The present search report has been drawn up for all claims

| Place of search | Date of completion of the search | Examiner |
|---|---|---|
| Munich | 3 March 2026 | Bîrlescu, V |

## ANNEX TO THE EUROPEAN SEARCH REPORT
## ON EUROPEAN PATENT APPLICATION NO.

EP 25 20 8757

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2026

| Patent document cited in search report | Publication date | Patent family member(s) | | Publication date |
|---|---|---|---|---|
| WO 2019160962 A1 | 22-08-2019 | CA | 3091176 A1 | 22-08-2019 |
| | | CN | 112534467 A | 19-03-2021 |
| | | EP | 3752977 A1 | 23-12-2020 |
| | | US | 2019251679 A1 | 15-08-2019 |
| | | US | 2021118106 A1 | 22-04-2021 |
| | | US | 2023072493 A1 | 09-03-2023 |
| | | WO | 2019160962 A1 | 22-08-2019 |
| WO 2008070683 A1 | 12-06-2008 | JP | 2010511486 A | 15-04-2010 |
| | | US | 2010073469 A1 | 25-03-2010 |
| | | US | 2013162944 A1 | 27-06-2013 |
| | | US | 2013169929 A1 | 04-07-2013 |
| | | WO | 2008070683 A1 | 12-06-2008 |

EPO FORM P0459

For more details about this annex : see Official Journal of the European Patent Office, No. 12/82